# EUROPEAN PATENT APPLICATION

(11) **EP 1 352 653 A1**
(43) Date of publication of application: **15.10.2003**
(21) Application number: 01940097.7
(22) Date of filing: 24.05.2001
(51) Int. Cl.: A61K 31/343, A61P 43/00

(54) **DIHYDROFURAN CYCLIC TANSHINTONES USED IN TREATING HYPERAMMONEMIA AND HEPATIC ENCEPHALOPATHY**

(30) Priority: 16.01.2001 CN 01107460
(71) Applicant: Zhongshan University, Guangdong 5102075 (CN); Guagzhou Medtech Zhongda Biotechnology Company, Ltd., Guangdong 5102075 (CN)
(72) Inventor: GU, Lianquan, Zhongshan University, Guangdong 510275 (CN); BU, Xianzhang, Zhongshan University, Guangdong 510275 (CN); MA, Lin, Zhongshan University, Guangdong 510275 (CN)
(74) Representative: Goddar, Heinz J., Dr.
(86) International application number: CN0100861
(87) International publication number: WO02055070

(57) **Abstract**

The present invention relates to use of dihydrofuran cyclic tanshinones represented by the formula (I): 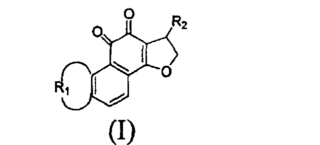 wherein R₁ is selected from a group consisting of -CH₂CH₂CH₂C(CH₃)₂-, -CHCHCHC(CH₃)-, -CH₂CH₂CHC(CH₃)-, or -CH₂CH₂CH₂C(CH₂)-, R₂ is H or C₁-C₃ alkyl etc. or a pharmaceutical composition containing the same for manufacturing medicine in preventing or treatment of hyperammonemia caused by chronic hepatitis and hepatocirrhosis, including hepatic encephalopathy and subclinical hepatic encephalopathy caused by hyperammonemia etc.

## Description

### Field of the Invention:

The present invention relates to use of dihydrofuran cyclic tanshinones, especially Cryptotanshinone and Dihydrotanshinone-I for manufacturing medicine in preventing or treating hyperammonemia (over concentration of ammonia and phenethylamine in the blood) caused by chronic hepatitis and hepatocirrhosis etc., especially hepatic encephalopathy (HE) and subclinical hepatic encephalopathy (SHE) caused by hyperammonemia, and so on.

### Background of the Invention:

Hyperammonemia is one of the major symptoms of chronic hepatitis and hepatocirrhosis. There are mainly two sources of ammonia production in the body: intestine and kidney. Most of the ammonia produced by them can be detoxicated through the ornithine cycle for synthesizing urea in the liver when its function is normal. However, the liver function of the chronic hepatitis and hepatocirrhosis patients is abnormal, and the ability of the liver to eliminate ammonia toxin is weakened, so there is increased amount of ammonia infiltrated into the blood, especially to the hepatocirrhosis patients for their greatly increased ammonia production caused by intestine congestion with portal vein diffluence formation and refluence blockage. Meanwhile, the kidney function of the chronic hepatitis and hepatocirrhosis patients is also impaired which also leads to apparently increased production of ammonia. Therefore, hyperammonemia is a symptom commonly shown by chronic hepatitis and hepatocirrhosis patients. Ammonia is a kind of basic toxic substance with poisoning effect to many major organs especially the brain. Long-term hyperammonemia is a major cause of aggravation and deterioration of chronic hepatitis and hepatocirrhosis, and initiation of hepatic encephalopathy (HE) and subclinical hepatic encephalopathy (SHE).

Hepatic encephalopathy (HE) is a syndrome of central nervous system dysfunction based on metabolizing disturbance. Its main clinical manifestation is consciousness disorder, behavior disorder and coma (Pathology, edited by Li Yu-Lin, People's Health Press, 2000, p340-350). There are primarily two mechanisms for hepatic encephalopathy (HE). The first mechanism is the high concentrated blood ammonia caused by liver dysfunction permeating through blood-brain barrier and entering into the brain tissue. The toxic effect of ammonia to brain tissue chiefly exists in its influence to the energy metabolism of brain cells, resulting in a decrease of the concentration of energy-rich phosphate compounds, some coenzymes and α-ketoglutarate. In normal situation, the liver can transform most of the ammonia produced in metabolism in the body into urea which is then removed form the kidney. In the situation of liver dysfunction, the ammonia eliminating ability will be weakened, thus the blood ammonia concentration increased. Moreover, at the time of alkalosis, the ionic type ammonium in the body will be transformed into molecular type ammonia, which also leads to a raise of blood ammonia concentration. The second mechanism is the action of the pseudo-neurotransmitter. The phenethylamine formed through decarboxylase effect by some amino acid such as phenylalanine can be decomposed and eliminated in the liver in normal situation, however, the blood phenethylamine concentration will rise and enter into the brain tissue through the blood-brain barrier, and converse into phenylethanol amine through the action of the enzyme in the neurocyte when the liver function is abnormal. Phenylethanol amine is a kind of pseudo-neurotransmitter with a similar structure to normal neurotransmitters such as dopamine, norepinephrine etc., but far weaker physiological functions. Phenylethanol amine can compete with normal neurotransmitter and so cause the dysfunction of cranial nerves.

Subclinical hepatic encephalopathy (SHE) is a kind of hepatic encephalopathy with intelligence, nerves or psyche defect manifested by liver dysfunction patients. Its symptoms are mental declining, performance lowering and neuro-response slowing. Its pathogenesis is similar to that of hepatic encephalopathy, which mainly caused by the raised concentration of blood ammonia and phenethylamine caused by the abnormal liver function of chronic hepatitis and cirrhosis patients. The more the severity of the liver function damage, the higher the incidence of the subclinical hepatic encephalopathy, and the easier the disease worsening into hepatic encephalopathy. According to the statistics, there are more than 50% of hepatocirrhosis patients with the symptoms of subclinical hepatic encephalopathy (Li Yu-Yuan etc., Chinese Journal of Internal Medicine, 2000, 39(9), 625)

Eliminating the highly concentrated ammonia and phenethylamine etc. can effectively ameliorate the symptoms of hepatic encephalopathy and subclinical hepatic encephalopathy. To date the drugs used to treat hyperammonemia, hepatic encephalopathy and subclinical hepatic encephalopathy such as lactulose, sodium glutamate and arginine etc. (Drugs, Wang Ru-Long and Yuan Zheng-Ping edited, chemical industry press, the third edition(1999), 655-665), have both their limitations, such as large dosage, needing of in-body enzyme participation when lowering blood ammonia, unsuitable to the patients with diabetes and kidney function failure, and their un-ideal effects etc..

Dan-shen is the root and rhizome of *Salvia miltiorrhiza* Bungev, family labiatae, bitter in taste and attributive to heart and liver channels. Dan-shen is widely used in clinics as a traditional Chinese herb. Its main indication is to disperse blood stasis and alleviate pain, to promote blood circulation and open channels, to clear away heat and relieve vexation, and so on. Recent studies showed that following major pharmacological actions exist in dan-shen and its extract.

1. It can promote the DNA synthesis in hepatocytes, and has protection effect to hepatocytes and acceleration to the regeneration of hepatocytes. It can alleviate the lung fibrosis and has anti-tuberculosis effect. 2. It can promote healing and has the effect of keeping the integrality of the gastric mucosal barrier and enhancing its defense function. 3. It can improve the induced renal failure, alleviate the uraemic symptoms of rat, and significantly reduce the blood concentration of urea nitrogen, methylguanidine and guanidino-succinic acid, and accelerate the recovery of the renal function. 4. It can dilate the coronary artery, increase its blood flow, prevent and treat myocardial infarct, and has the effect of anti-oxygen free radicals, blood circulation promotion and blood stasis dispersion, platelet-suppression, and fibrinolysis activity-promotion. 5. It can relieve pain and has suppression effects on central nervous system, and has the effect of anti-inflammation, anti-bacteria, and anti-tumor etc.

However, to present there is no report on the preventing and treating of the hyperammonemia caused by chronic hepatitis and hepatocirrhosis, hepatic encephalopathy and subclinical hepatic encephalopathy caused by hyperammonemia etc. using the extract of dan-shen, especially the tanshinonic compounds with dihydro-furan nucleus structure.

The object of the present invention is to find and develop the new pharmaceutical use of the dan-shen extract especially the tanshinonic compounds with dihydro-furan nucleus structure.

Through investigation the inventor unexpectedly found that good effect existed in the preventing and treating of the hyperammonemia caused by chronic hepatitis and hepatocirrhosis, hepatic encephalopathy and subclinical hepatic encephalopathy caused by hyperammonemia etc. using tanshinonic compounds with dihydro-furan nucleus structure extracted from dan-shen.

### Summary of the Invention:

The present invention provides the use of dihydrofuran cyclic tanshinones represented by the formula (I): 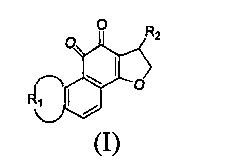 wherein R₁ represents -CH₂CH₂CH₂C(CH₃)₂-, -CHCHCHC(CH₃)-, - CH₂CH₂CHC(CH₃)-, or -CH₂CH₂CH₂C(CH₂)-, R₂ represents H or C₁-C₃ alkyl, or a composition containing one of them for manufacturing medicine in preventing or treating hyperammonemia caused by chronic hepatitis and hepatocirrhosis, including hepatic encephalopathy and subclinical hepatic encephalopathy caused by hyperammonemia.

In a preferred embodiment of the invention, wherein R₁ is selected from a group consisting of -CH₂CH₂CH₂C(CH₃)₂-, -CHCHCHC(CH₃)-, - CH₂CH₂CHC(CH₃)-, and -CH₂CH₂CH₂C(CH₂)-, and R₂ is methyl.

In a preferred embodiment of the invention, R₁ is selected from the group consisting of -CH₂CH₂CH₂C(CH₃)₂-, and -CHCHCHC(CH₃)-, and R₂ is methyl.

In a preferred embodiment of the invention, said compound represented by formula (I) is selected from the group consisting of Cryptotanshinone (1,6,6-trimethyl-1,2,6,7,8,9-hexahydrophenanthrene [1,2-b] furan-10,11-dione) and Dihydrotanshinone I (1,6-dimethyl-1,2-dihydrophenanthrene[1,2-b]furan-10, 11-dione).

The present invention also provides a pharmaceutical composition for preventing or treating hyperammonemia caused by chronic hepatitis and hepatocirrhosis, including hepatic encephalopathy and subclinical hepatic encephalopathy caused by hyperammonemia, which comprises the compound represented by the formula (I) and optionally a pharmaceutically acceptable diluent or vehicle.

The present invention further provides a method for preparing a pharmaceutical composition for preventing or treating hyperammonemia caused by chronic hepatitis and hepatocirrhosis, including hepatic encephalopathy and subclinical hepatic encephalopathy caused by hyperammonemia, comprising formulating a compound represented by the formula (I) optionally with a pharmaceutically acceptable diluent or vehicle.

The present invention further provides a method for preventing or treating hyperammonemia caused by chronic hepatitis and hepatocirrhosis, including hepatic encephalopathy and subclinical hepatic encephalopathy caused by hyperammonemia, comprising administering to the patient an effective amount of a compound represented by the formula (I) or a pharmaceutical composition containing the same.

According to the present invention, the compound represented by the formula (I) or its pharmaceutical composition can be prepared by the method well-known in this field and can be delivered through oral, parenteral (such as intravenous injection, intra-muscular injection and hypodermic injection etc.) or local (such as sublingual, urethral and rectal medication etc.) administration. The oral medication includes the form of tablet, chewing, capsule, pill, suspension, emulsion and solution etc. Parenteral medication includes the injection and local administration includes formulations such as cream, ointment, sticking, suppository and spray etc.

### Detailed Description of the Invention

The investigations taken by the inventor showed that the dihydrofuran cyclic tanshinones such as Cryptotanshinone and Dihydrotanshinone I etc. can react with ammonia, methylamine, ethylamine and phenethylamine in a mild condition and form new tanshinonic derivative, which suggests that these compounds have special therapeutical effect on the preventing and treating of the hyperammonemia caused by chronic hepatitis and hepatocirrhosis, and hepatic encephalopathy and subclinical hepatic encephalopathy caused by hyperammonemia etc. The reaction formula is shown as follow in which the meaning of R₁ and R₂ is just as defined above.

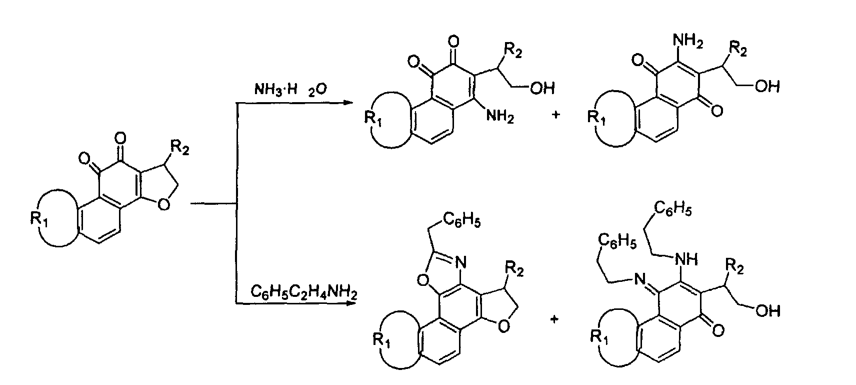

The tanshinonic compounds with dihydro-furan nucleus structure used in the present invention can be obtained from the Chinese herb dan-shen through solvent extraction method or from chemical synthesis. The extraction method is generally used to achieve the compounds in the present invention considering the factors of cost etc. for the abundant resources of dan-shen.

In the present invention, we confirmed through chemical reaction experiments that the dihydrofuran cyclic tanshinones such as Cryptotanshinone and Dihydrotanshinone I etc. can react with ammonia, methylamine, ethylamine and phenethylamine in a mild condition and form new tanshinonic derivative, which suggests that these compounds have the potential to be used to reduce the toxic nitrogenous metabolite in the body such as high concentrate ammonia and phenethylamine etc.

The present invention demonstrated through hyperammonemia animal model experiments that the dihydrofuran cyclic tanshinones such as Cryptotanshinone and Dihydrotanshinone I etc. can effectively reduce the blood ammonia concentration of hyperammonemia rats, shorten the hepatic coma induced by hyperammonemia and decrease the mortality, which suggested that these compounds can be effective drugs to prevent and treat hyperammonemia caused by chronic hepatitis and hepatocirrhosis, hepatic encephalopathy and subclinical hepatic encephalopathy caused by hyperammonemia, and etc..

The present invention determined through toxicologic study in rats that the dihydrofuran cyclic tanshinones such as Cryptotanshinone and Dihydrotanshinone I etc. are drugs without any toxic and side effect and can be used safely.

Because the Chinese herb dan-shen has good promotion and recovery effect to liver and kidney function, the pharmaceutical agents in the present invention majoring with Dihydrotanshinonic compounds of the main active ingredients in dan-shen not only can effectively reduce the blood ammonia and phenethylamine concentration thus improving the symptoms of chronic hepatitis, cirrhosiss, hepatic encephalopathy and subclinical hepatic encephalopathy, but also can effectively accelerate the recovering of the liver function, thus posses ideal therapeutic effects to chronic hepatitis, cirrhosiss, hepatic encephalopathy and subclinical hepatic encephalopathy.

Followings are examples further describing the present invention.

### Example 1:

### Extraction, isolation and purification of Dihydrotanshinone I:

Dried dan-shen 1 kg was extracted 3 times 24 h each using 1500 mL 95% alcohol, then vacuum concentrated to 500 ml, and added 500 ml water, then extracted 4 times using 1000 ml chloroform, then the extract was vacuum concentrated, and isolated through gradient elution by column chromatography, with silica gel of 100-200 mesh, and eluent as petroleum ether / acetic ester mixture containing 1%-20% acetic ester. About 0.12g Dihydrotanshinone I was obtained.

### Example 2:

### Extraction, isolation and purification of Cryptotanshinone from dan-shen:

Dried dan-shen 1 kg was extracted 3 times 24 h each using 1500 mL 95% alcohol, then vacuum concentrated to 500 ml, and 500 ml water was added, and then extracted 4 times using 1000 ml chloroform, then the extract was vacuum concentrated, and isolated through gradient elution by column chromatography, with silica gel of 100-200 mesh, and eluent as petroleum ether / acetic ester mixture containing 10%-50% acetic ester. About 0.35g Cryptotanshinone obtained.

### Example 3:

### In vitro reaction of Dihydrotanshinone I and ammonia:

0.5 mmol ammonia and 30 ml water was added into a test tube, and then added 0.5 mmol Dihydrotanshinone I in 1.0 ml alcohol, and vibrated 2-8 h in 37±5°C, then monitored by TLC and isolated by column chromatography, and obtained the products of Dihydrotanshinone I and ammonia reaction as 1-amino-2-(1'-hydroxy-2'-propyl)-8-methyl-phenanthrene-3,4-dione and 3-amino-2-(1'-hydroxy-2'-propyl)-8-methyl-phenanthrene-1,4-dione. The structure of products has been confirmed by ¹³C-¹HNMR, MS, and elementary analysis, which showed that Dihydrotanshinone I can react with ammonia in vitro and thus lower the ammonia concentration.

### Example 4:

### In vitro reaction of Dihydrotanshinone I and phenethylamine:

0.5 mmol phenethylamine and 30 ml water was added into a test tube, and then added 0.5 mmol Dihydrotanshinone I in 1.0 ml alcohol, and vibrated 2-8 h in 37 ±5°C, then monitored by TLC and isolated by column chromatography, and obtained the products of Dihydrotanshinone I and phenethylamine reaction as 2-benzyl-4,9-dimethyl-4,5-dihydro-1,6-dioxa-3-azo-dicyclopenta [a,c] phenanthrene and 2-(1'-hydroxy-2'-propyl)-8-methyl-3-phenethylamino-4-phenethylimino-4H-phenanthren-1-one. The structure of products has been confirmed by ¹³C-¹HNMR, MS, and elementary analysis, which showed that Dihydrotanshinone I can react with phenethylamine in vitro and thus lower the phenethylamine concentration.

### Example 5:

### In vitro reaction of Cryptotanshinone and ammonia:

0.5 mmol ammonia and 30 ml water was added into a test tube, and then added 0.5 mmol Cryptotanshinone in 1.0 ml alcohol, and vibrated 0.5-3 h in 37±5°C, then monitored by TLC and isolated by column chromatography, and obtained the products of Cryptotanshinone and ammonia reaction as 1-amino-2-(1'-hydroxy-2 '-propyl)-8,8-dimethyl-5,6,7,8-tetrahydro-phenanthrene -3,4-dione and 3-amino-2-(1'-hydroxy-2'-propyl)-8-methyl-phenanthrene-1,4-dione. The products structure has been confirmed by ¹³C-¹HNMR, MS, and elementary analysis, which showed that Cryptotanshinone can react with ammonia in vitro and thus lower the ammonia concentration.

### Example 6:

### In vitro reaction of Cryptotanshinone and phenethylamine:

0.5 mmol phenethylamine and 30 ml water was added into a test tube, and then added 0.5 mmol Cryptotanshinone in 1.0 ml alcohol, and vibrated 0.5-3 h in 37 ± 5°C, then monitored by TLC and isolated by column chromatography, and obtained the products of Cryptotanshinone and phenethylamine reaction as 2-benzyl-4,9,9-trimethyl-4,5,9,10,11,12-hexahydro-1,6-dioxa-3-azo-dicyclopenta [a,c]phenanthrene and 2-(1'-hydroxy-2'-propyl)-8,8-dimethyl-3-phenethylamino -4-phenethylimino-5,6,7,8-tetrahydro-4H-phenanthren-1-one. The structure of products has been confirmed by ¹³C-¹HNMR, MS, and elementary analysis, which showed that Cryptotanshinone can react with phenethylamine in vitro and thus lower the phenethylamine concentration.

### Example 7:

### In vitro reaction of tanshinone IIA (one of dihydrofuran tanshinones) and ammonia:

No apparent reaction showed in 48h monitoring with TLC according to the method of Example 3 and substituting Dihydrotanshinone I with tanshinone IIA, which suggested that no interaction exists in this condition between tanshinone IIA and ammonia.

### Example 8:

### In vitro reaction of tanshinone IIA and phenethylamine:

No apparent reaction showed in 48h monitoring with TLC according to the method of Example 4 and substituting Dihydrotanshinone I with tanshinone IIA, which suggested that no interaction exists in this condition between tanshinone IIA and a phenethylamine.

### Example 9:

### Blood ammonia reducing experiment of Dihydrotanshinone I and tanshinone IIA(in injection):

The animals used in this experiment were 50 healthy male SD rats weighing 250-300g. They were randomly divided into 5 groups, that is, group of normal control, experimental control (acetamide group), sodium glutamate/acetamide group, Dihydrotanshinone I/acetamide group and tanshinone IIA/acetamide group. The rats in all those groups except the normal control group were injected intraperitoneally with saline, sodium glutamate injection (with a dose of 410 mg/kg), saline solution of Dihydrotanshinone I (containing small amount of surface active agent, with a dose of 10 mg/kg), saline solution of tanshinone IIA (containing small amount of surface active agent, with a dose of 10 mg/kg) respectively. 45 min after the injection, the rats in all those groups except control group were injected intraperitoneally with 5.5 mmol/kg acetamide. Above injections continued for 4 days. In the 4^{th} day, 30 min after the acetamide injection, blood samples of the rats in each group were collected by eyeball removing method immediately, and processed with the anticoagulant EDTA-Na, then the blood ammonia concentration was determined using conventional method. The results were showed in table 1. The dose of acetamide in this example and following examples was settled with the reference of its actual clinical dose. The method for determining the blood ammonia concentration in this example and following examples was enzyme-UV method, the instrument used was HITACHI-7170 automatic analyzer, and the kit used was AMMONIA kit.

**Table 1**

| Groups | Blood ammonia concentration (µmol/L) | Reduction rate (%) |
|---|---|---|
| Normal control group | 115 | --- |
| Experimental control group | 885 | --- |
| Sodium Glutamate group | 480 | 53 |
| Dihydrotanshinone I group | 490 | 51 |
| Tanshinone IIA group | 850 | <5 |

With the same experiment method, 90 min after the acetamide injection at the 4^{th} day, blood samples of the rats in each group were collected by eyeball removing method immediately, and processed with the anticoagulant EDTA-Na, then the blood ammonia concentration was determined. The results were showed in table 2.

**Table 2**

| Groups | Blood ammonia concentration (µmol/L) | Reduction rate (%) |
|---|---|---|
| Normal control group | 115 | --- |
| Experimental control group | 285 | --- |
| Sodium Glutamate group | 120 | 97 |
| Dihydrotanshinone I group | 125 | 96 |
| Tanshinone IIA group | 265 | <12 |

### Example 10

### Blood ammonia reducing experiment of Cryptotanshinone (in injection):

The animals used in this experiment were 40 healthy male SD rats weighing 250-300g. They were randomly divided into 4 groups, that is, normal control group, experimental control group (acetamide group), sodium glutamate/acetamide group, and Cryptotanshinone/acetamide group. The rats in all those groups except the normal control group were injected intraperitoneally with saline, sodium glutamate injection (with a dose of 410 mg/kg), and saline solution of Cryptotanshinone (containing small amount of surface active agent, with a dose of 10 mg/kg) respectively. 45 min after the injection, the rats in all those groups except the normal control group were injected intraperitoneally with 5.5 mmol/kg acetamide. Above injections continued for 4 days. In the 4^{th} day, 30 min after the acetamide injection, blood samples of the rats in each group were collected by eyeball removing method immediately, and processed with the anticoagulant EDTA-Na, then the blood ammonia concentration was determined. The results were showed in table 3.

**Table 3**

| Groups | Blood ammonia concentration (µmol/L) | Reduction rate (%) |
|---|---|---|
| Normal control group | 115 | --- |
| Experimental control group | 885 | --- |
| Sodium Glutamate group | 480 | 53 |
| Cryptotanshinone group | 420 | 60 |

With the same experiment method, 90 min after the acetamide injection at the 4^{th} day, blood samples of the rats in each group were collected by eyeball removing method immediately, and processed with the anticoagulant EDTA-Na, then the blood ammonia concentration was determined. The results were showed in table 4.

**Table 4**

| Groups | Blood ammonia concentration (µmol/L) | Reduction rate (%) |
|---|---|---|
| Normal control group | 116 | --- |
| Experimental control group | 285 | --- |
| Sodium Glutamate group | 110 | 100 |
| Cryptotanshinone group | 110 | 100 |

### Example 11:

### Blood ammonia reducing experiment of Dihydrotanshinone I and tanshinone IIA (in oral):

The same experiment condition in example 9 was adopted except the changes of Dihydrotanshinone I injecting into intragastric (i.g.) application (the dose is 100 mg/kg), and tanshinone IIA injecting into intragastric application (the dose is 100 mg/kg). 120min after the intragastric application, the rats in all those groups except control group were injected intraperitoneally with acetamide. In the 4^{th} day, 30 min after the acetamide injection, blood samples of the rats in each group were collected by eyeball removing method immediately, and processed with the anticoagulant EDTA-Na, then the blood ammonia concentration was determined. The results were showed in table 5.

**Table 5**

| Groups | Blood ammonia concentration (µmol/L) | Reduction rate (%) |
|---|---|---|
| Normal control group | 115 | --- |
| Experimental control group | 885 | --- |
| Sodium Glutamate group | 510 | 49 |
| Dihydrotanshinone I group | 550 | 44 |
| Tanshinone IIA group | 865 | <4 |

With the same experiment method, 90 min after the acetamide injection at the 4^{th} day, blood samples of the rats in each group were collected by eyeball removing method immediately, and processed with the anticoagulant EDTA-Na, then the blood ammonia concentration was determined. The results were showed in table 6.

**Table 6**

| Groups | Blood ammonia concentration (µmol/L) | Reduction rate (%) |
|---|---|---|
| Normal control group | 115 | --- |
| Experimental control group | 285 | --- |
| Sodium Glutamate group | 130 | 91 |
| Dihydrotanshinone I group | 135 | 89 |
| Tanshinone IIA group | 270 | <10 |

### Example 12:

### Blood ammonia reducing experiment of Cryptotanshinone (in oral):

The same experiment condition in example 9 was adopted except the changes of Dihydrotanshinone I injecting into intragastric (i.g.) application (the dose is 100 mg/kg), and tanshinone IIA injecting into intragastric application (the dose is 100 mg/kg). 120min after the intragastric application, the rats in all those groups except control group were injected intraperitoneally with acetamide. In the 4^{th} day, 30 min after the acetamide injection, blood samples of the rats in each group were collected by eyeball removing method immediately, and processed with the anticoagulant EDTA-Na, then the blood ammonia concentration was determined. The results were showed in table 7.

**Table 7**

| Groups | Blood ammonia concentration (µmol/L) | Reduction rate (%) |
|---|---|---|
| Normal control group | 115 | --- |
| Experimental control group | 885 | --- |
| Sodium Glutamate group | 510 | 49 |
| Cryptotanshinone group | 470 | 54 |

With the same experiment method, 90 min after the acetamide injection at the 4^{th} day, blood samples of the rats in each group were collected by eyeball removing method immediately, and processed with the anticoagulant EDTA-Na, then the blood ammonia concentration was determined. The results were showed in table 8.

**Table 8**

| Groups | Blood ammonia concentration ( µ mol/L) | Reduction rate (%) |
|---|---|---|
| Normal control group | 115 | --- |
| Experimental control group | 285 | --- |
| Sodium Glutamate group | 130 | 91 |
| Cryptotanshinone group | 120 | 97 |

### Example 13

### Effect of Cryptotanshinone (in injection) on the occurrence of coma and death caused by acute hepatocirrhosis in rats:

The animals used in this experiment were 40 healthy male SD rats weighing 250-300g. They were randomly divided into 4 groups, that is, group of normal control, experimental control (acetamide group), sodium glutamate/acetamide group, and Cryptotanshinone/acetamide group. The rats in all those groups except the normal control group were injected intraperitoneally with saline, sodium glutamate injection (with a dose of 410 mg/kg), and saline solution of Cryptotanshinone (containing small amount of surface active agent, with a dose of 10 mg/kg) respectively. 45 min after the injection, the rats in all those groups except the normal control group were injected intraperitoneally with peanut oil containing 0.1% tetrachloro-methane. Above injections continued for 4 days. In the 4^{th} day, 45 min after the tetrachloride peanut oil injection, the rats in each group (including the normal control group) were injected intraperitoneally with 8.5 mmol/kg acetamide solution. The time from acetamide injection to coma (latent period), from coma to awakening (coma time) and death rate in rats was recorded. The results were showed in table 9.

**Table 9**

| Groups | Latent period (min) | Coma time (min) | Mortality (%) |
|---|---|---|---|
| Normal control group | 9 | 115 | 60 |
| Experimental control group | 6 | 135 | 70 |
| Sodium Glutamate group | 11 | 75 | 50 |
| Cryptotanshinone group | 12 | 65 | 20 |

### Example 14:

### Effect of Cryptotanshinone (in oral) on the occurrence of coma and death caused by acute hepatocirrhosis in rats:

The same experiment condition in example 13 was adopted except the changes of acetamide injecting into acetamide oral taking (the dose was 200 mg/kg), and Cryptotanshinone injecting into oral taking (the dose is 100 mg/kg). In the 4^{th} day, 45 min after the tetrachloride peanut oil injection, the rats in each group (including the normal control group) were injected intraperitoneally with 8.5 mmol/kg acetamide solution. The time from acetamide injection to coma (latent period), from coma to awakening (coma time) and death rate in rats was recorded. The results were showed in table 10.

**Table 10**

| Groups | Latent period (min) | Coma time (min) | Mortality (%) |
|---|---|---|---|
| Normal control group | 9 | 115 | 60 |
| Experimental control group | 6 | 135 | 70 |
| Sodium Glutamate group | 9 | 90 | 50 |
| Cryptotanshinone group | 12 | 75 | 20 |

### Example 15:

### Toxicity test of Cryptotanshinone:

Acute toxicity test in mouse: The animals used in this study were healthy male mice. The drug was administrated intragastrically for 7 days once daily. Still no toxic symptom or death showed in the experiment mice when the administrated dose added to 1.5 g/kg. The experiment showed that LD₅₀ of Cryptotanshinone >> 1.5 g / kg.

30 days toxicity test in muse: The animals used in this study were healthy male mice. The drug was continually administrated intragastrically for 30 days with dose of 200 mg/kg day. The result showed that no death of mice manifested and no apparent abnormal change or toxic reaction in the growth and development, hematopoietic function, and biochemical index of mice existed. No pathology change of major organs was found by anatomical inspection and tissue microscopy inspection.

### Industrial applicability

Above examples suggested that:
(1) The dihydrofuran cyclic tanshinones represented by the formula (I) in the present invention can react with ammonia or phenethylamine in physiologic condition and thus achieve the aim of ammonia or phenethylamine cleaning.
(2) The experiment on the hyperammnonemia animal model showed that the dihydrofuran cyclic tanshinones represented by the formula (I) in the present invention have good function to reduce the blood ammonia concentration, while the tanshinonic compounds with other structure characteristics such as tanshinone IIA have no such function.
(3) The experiment on the acute hepatocirrhosis hyperammnonemia rat model showed that the dihydrofuran cyclic tanshinones represented by the formula (I) in the present invention can effectively prolong the latent phase of hepatic coma induced by hyperammnonemia, shorten the time of hepatic coma, and effectively reduce the death rate.
(4) The toxicity test of Cryptotanshinone (a kind of dihydrofuran cyclic tanshinones represented by the formula (I) in the present invention) indicated that it is safe to use as drug in normal dosage.
(5) Compared with glutamic acid which is a typical drug commonly used to treat hyperammonemia, hepatic encephalopathy (HE) and subclinical hepatic encephalopathy (SHE) in the art, the same or more effective result can be obtained from the compounds in the present invention with even smaller dosage. The therapeutic efficacy of the compounds in the present invention is more significant than glutamic acid especially when the liver function is damaged.

Without being bound by theory, it is believed that because there is no need of enzyme participating in the reaction clearing blood ammonia and phenethylamine by the dihydrofuran cyclic tanshinones represented by the formula (I) in the present invention, the function of its clearing blood ammonia and phenethylamine is independent of whether the liver function is normal or not. However, glutamic acid commonly used in the art needs the participation of enzymes during blood ammonia reduction, so it can't exert its function when the liver function is abnormal. Therefore, the compounds in the present invention have wider applicability and more effectiveness. So, the dihydrofuran cyclic tanshinones represented by the formula (I) in the present invention can be used to prepare effective drugs to prevent and treat hyperammonemia caused by chronic hepatitis and hepatocirrhosis, hepatic encephalopathy (HE) and subclinical hepatic encephalopathy (SHE) caused by hyperammonemia, and so on.

## Claims

1. Use of dihydrofuran cyclic tanshinones represented by the formula (I): 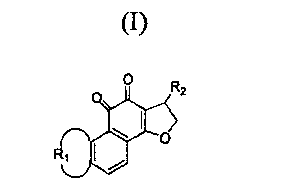 wherein R₁ represents -CH₂CH₂CH₂C(CH₃)₂-, -CHCHCHC(CH₃)-, -CH₂CH₂CHC(CH₃)-, or -CH₂CH₂CH₂C(CH₂)-, R₂ represents H or C₁-C₃ alkyl, or a composition containing one of them for manufacturing medicine in preventing or treating of hyperammonemia caused by chronic hepatitis and hepatocirrhosis including hepatic encephalopathy and subclinical hepatic encephalopathy caused by hyperammonemia.

2. The use of claim 1, wherein R₁ is selected from the group consisting of -CH₂CH₂CH₂C(CH₃)₂-, -CHCHCHC(CH₃)-, -CH₂CH₂CHC(CH₃)-, and -CH₂CH₂CH₂C(CH₂)-, and R₂ is methyl.

3. The use of claim 1, wherein R₁ is selected from the group consisting of -CH₂CH₂CH₂C(CH₃)₂-, and -CHCHCHC(CHₛ)-, and R₂ is methyl.

4. The use of claim 1, wherein said compound represented by formula (I) is selected from the group consisting of Cryptotanshinone and Dihydrotanshinone I.

5. A pharmaceutical composition for preventing or treating hyperammonemia caused by chronic hepatitis and hepatocirrhosis, including hepatic encephalopathy and subclinical hepatic encephalopathy caused by hyperammonemia, which comprises the compound represented by the formula (I) according to any one of claims 1 to 4 and an optionally pharmaceutically acceptable diluent or vehicle.

6. A method for preparing a pharmaceutical composition for preventing or treating hyperammonemia caused by chronic hepatitis and hepatocirrhosis, including hepatic encephalopathy and subclinical hepatic encephalopathy caused by hyperammonemia, which comprises formulating the compound represented by the formula (I) according to any one of claims 1 to 4 with an optionally pharmaceutically acceptable diluent or vehicle.

7. A method for preventing or treating hyperammonemia caused by chronic hepatitis and hepatocirrhosis, including hepatic encephalopathy and subclinical hepatic encephalopathy caused by hyperammonemia, which comprises using effective amount of the compound represented by the formula (I) or the pharmaceutical composition containing the same according to any one of claims 1 to 4 to treat the patients.

8. The use of any one of claims 1 to 4, wherein said medicine is administered in a form of injection, tablet, pill, capsule, solution, suspension, emulsion, cream, ointment, spray, chewing, suppository or paste.

9. The use of any one of claims 1 to 4, wherein said medicine is administered to a patient by a route of oral, sublingual, intravenous, intra-muscular, subcutaneous or rectal.
